# EUROPÄISCHE PATENTSCHRIFT

(11) **EP 3 544 525 B1**
(45) Veröffentlichungstag und Bekanntmachung des Hinweises auf die Patenterteilung: **29.12.2021**
(21) Anmeldenummer: 17807758.2
(22) Anmeldetag: 15.11.2017
(51) Int. Cl.: A61B 17/08, A61B 90/00, A61B 17/02

(54) **INSTRUMENT ZUM DEHNEN DER HAUT**
INSTRUMENT FOR SKIN STRETCHING
INSTRUMENT POUR DILATER LA PEAU

(30) Priorität: 23.11.2016 DE 102016122593
(43) Veröffentlichungstag der Anmeldung: 02.10.2019
(73) Patentinhaber: Biowim Products GmbH, 79199 Kirchzarten (DE)
(72) Erfinder: FLEISCHMANN, Wilhelm, 79104 Freiburg (DE)
(74) Vertreter: Westphal, Mussgnug & Partner Patentanwälte mbB
(86) Internationale Anmeldenummer: PCT/EP2017/079341
(87) Internationale Veröffentlichungsnummer: WO 2018/095784

(56) Entgegenhaltungen:
- EP-B1- 2 651 313
- US-A1- 2009 227 845
- US-A1- 2010 113 885
- US-A1- 2011 009 706

## Beschreibung

Die Erfindung betrifft ein Instrument zum Dehnen der Haut gemäß Oberbegriff des Patentanspruchs 1.

Zum Schließen großflächiger Hautdefekte ist es bekannt, die Haut insbesondere im Bereich der Wundränder zu dehnen (Skin Stretching), um die Wundränder gegeneinander zu ziehen. Durch die über einen definierten Zeitraum auf die Haut wirkende Zugkraft wird diese über ihre natürliche Elastizitätsgrenze hinaus gedehnt, wobei die Dehnung eine Flächenzunahme der Haut, eine Umverteilung von Gewebeflüssigkeit (antiödematöse Wirkung)sowie eine Steigerung der Zellproliferation und Matrixsynthese bewirkt. Die Flächenvergrößerung der Haut ermöglicht den Verschluss der Wunde und deren Abheilen wird durch die dehnungsinduzierte Stimulation der Wundheilung gefördert.

Für diese Technik des Skin Stretching sind Instrumente bekannt, bei denen wenigstens zwei Module auf einer Führung gegeneinander bewegbar angeordnet sind. Die Module weisen Verankerungsmittel auf, mit welchen sie jeweils auf der Haut fixiert werden können. An den einander gegenüberliegenden Wundrändern wird jeweils ein Modul auf der Haut verankert. Die Module werden auf der Führung gegeneinander bewegt, um die Wundränder zusammenzuziehen. Dabei wird auf die Haut außerhalb der Module eine Zugkraft ausgeübt, die die Haut dehnt. Ein Richtgesperre ermöglicht eine Bewegung der Module in der gewünschten Richtung zur Dehnung der Haut und blockiert eine entgegengesetzte Rückwärtsbewegung unter der elastischen Zugkraft der gedehnten Haut.

Ein Instrument dieser Gattung ist beispielsweise aus der EP 2 651 313 B1 bekannt. Bei diesem Instrument sind die Module auf einer langgestreckten formstabilen Führung verschiebbar. Das Richtgesperre wird gebildet durch eine an dem jeweiligen Modul angeordnete Sperrklinke, die in eine an der Führung ausgebildete Zahnung eingreift. Die Zahnung ist als Sägezahnung ausgebildet, deren schräg ansteigende Zahnflanke die Sperrklinke aus der Zahnung heraushebt, wenn das Modul in der gewünschten Richtung verschoben wird. Eine Rückwärtsbewegung unter der Zugkraft der Haut wird dadurch verhindert, dass die Sperrklinke gegen die jeweilige senkrechte Stützfläche der Sägezähne läuft. Dieses bekannte Instrument eignet sich nur dazu, die einander gegenüberliegenden Wundränder zusammenzuziehen. Als weiterer Stand der Technik wird die US 2010/0113885 A1 genannt.

Der Erfindung liegt die Aufgabe zugrunde, das Instrument der eingangs genannten Gattung so weiterzubilden, dass eine vielseitigere Verwendung für die Hautdehnung ermöglicht wird.

Diese Aufgabe wird erfindungsgemäß gelöst durch ein Instrument mit den Merkmalen des Patentanspruchs 1.

Vorteilhafte Ausführungen der Erfindung sind in den Unteransprüchen angegeben.

Der wesentliche Gedanke der Erfindung besteht darin, bei wenigstens einem auf der Führung bewegbaren Modul das Richtgesperre umkehrbar auszubilden. Dadurch können die Richtung der freien Bewegbarkeit des Moduls und die Sperrrichtung, in welcher die Bewegung des Moduls blockiert wird, jeweils um 180° umgekehrt werden, so dass die Bewegungsrichtung und die Sperrrichtung gegeneinander vertauscht werden.

Hierdurch wird es möglich, mit demselben Instrument unterschiedliche Dehnungswirkungen auf die Haut auszuüben. Ist das Richtgesperre des bewegbaren Moduls so eingestellt, dass zwei Module gegeneinander bewegt werden können, so kann das Instrument dazu verwendet werden, in herkömmlicher Weise die Haut der Wundränder gegeneinander zusammenzuziehen. Ist das Richtgesperre des bewegbaren Moduls so umgekehrt eingestellt, dass sich dieses bewegbare Modul nur von dem jeweils anderen Modul wegbewegen kann, eine Bewegung auf das andere Modul hin jedoch blockiert wird, so kann das Instrument dazu verwendet werden, auf eine gesunde Hautpartie zwischen diesen Modulen eine diese Hautpartie dehnende und streckende Zugkraft auszuüben. Dadurch kann ein zusätzlicher Hautgewinn erzielt werden, der insbesondere auch für einen Wundverschluss benutzt werden kann, ohne dass hierbei eine zusätzliche Zugbeanspruchung der Wundränder erforderlich ist.

In einer vorteilhaften Ausführung des umkehrbaren Richtgesperres ist eine in Längsrichtung der Führung verlaufende Zahnung mit quer zur Längsrichtung verlaufenden Rippen ausgebildet, die an ihren beiden in diesen entgegengesetzten Richtungen weisenden Flanken als senkrechte Stützfläche ausgebildet sind. In diese Zahnung greift eine an dem jeweiligen Modul angeordnete Sperrklinke ein, deren in die Zahnung eingreifende Spitze mit einer in die zulässige Bewegungsrichtung weisenden Abschrägung ausgebildet ist. Bewegt sich das Modul in der Bewegungsrichtung, so läuft die Sperrklinke mit dieser Abschrägung über die Rippen der Zahnung und wird durch diese Rippen aus der Zahnung herausgehoben. In der entgegengesetzten Sperrrichtung läuft die Spitze der Sperrklinke gegen die senkrechte Stützfläche der Rippen, so dass die Bewegung in Sperrrichtung blockiert wird. Eine Umkehr des Richtgesperres wird dadurch bewirkt, dass die Sperrklinke mit ihrer Abschrägung um 180° gedreht an dem Modul zum Einsatz kommt. Dies kann gegebenenfalls dadurch erreicht werden, dass an dem Modul zwei Sperrklinken angeordnet sind, deren abgeschrägte Spitzen um 180° gegeneinander gerichtet sind. Je nach der gewünschten Bewegungsrichtung wird eine der Sperrklinken aus der Zahnung herausgehoben und verriegelt, so dass diese Sperrklinke außer Funktion ist und nur die jeweils andere Sperrklinke die Bewegungsrichtung und die Sperrrichtung bestimmt.

In einer fertigungstechnisch einfachen Ausführung weist das Modul nur eine Sperrklinke auf, die die Bewegungsrichtung und die Sperrrichtung bestimmt. Das Modul kann dabei in zwei um 180° gedrehten Stellungen auf die Führung gesetzt werden, wodurch sich die Richtung und die Funktion der Sperrklinke umkehrt.

Weist das Instrument nur zwei Module auf, von denen wenigstens eines bewegbar und mit einem umkehrbaren Richtgesperre ausgebildet ist, so kann das Instrument einerseits zum Zusammenziehen von Wundrändern verwendet werden und andererseits durch Umkehr des Richtgesperres zum Dehnen eines gesunden Hautareals.

Bei einem Instrument mit wenigstens drei Modulen ergeben sich weitere Verwendungsmöglichkeiten. Zwei Module, von denen wenigstens eines bewegbar und mit einem Richtgesperre ausgebildet ist, können gegeneinander bewegt werden, um die Ränder einer zwischen diesen Modulen liegenden Wunde zusammenzuziehen. Ein drittes bewegliches Modul mit Richtgesperre kann so auf der Führung angeordnet werden, dass es von dem am Wundrand verankerten Modul wegbewegt wird, wodurch die Haut außerhalb des Wundrandes gedehnt wird, um eine zusätzliche Haut-Proliferation zu bewirken. Durch Umkehr des Richtgesperres kann die Bewegung auch in Richtung auf das am Wundrand verankerte Modul ermöglicht werden. Dadurch kann auf die Haut außerhalb des Wundrandes eine zusätzliche Zugkraft ausgeübt werden, durch welche gesunde Haut zusätzlich zu dem Wundrand hin nachgeschoben wird.

In einer weiteren Ausführung werden zwei oder mehr Module so auf einem Rahmen angebracht, dass die jeweilige Ausrichtung des Richtgesperres um 180 Grad verändert werden kann. Alternativ kann ein Modul auch zwei gegenläufig ausgerichtete Sperrklinken aufweisen, wobei je nach Bedarf, die Verriegelungsrichtung durch das Einrasten der entsprechenden Sperrklinke festgelegt wird. Die Führungen lassen sich nun in einer Bewegungsrichtung durch die am Rahmen befestigten Module ziehen, während die Bewegung in Gegenrichtung durch das Richtgesperre blockiert wird. Durch Wenden der am Rahmen befestigten Module um 180 Grad oder Aktivieren der entsprechenden doppelten Sperrklinke am Modul lässt sich die Richtung der freien Bewegung und der Blockade der jeweils zugeordneten Führung umkehren. Der Vorteil einer solchen Ausführung liegt zum einen darin, dass beim Zusammenschieben der Wundränder vom Rahmen aus, die vollständige Sichtbarkeit der Wunde innerhalb des Rahmens erhalten bleibt, da keine Führung die Wunde überkreuzt. Zum anderen lässt sich die innerhalb des Rahmens befindliche Haut in zwei Richtungen (z.B. bei einem rechteckigen Rahmen) oder sogar kreisförmig (z.B. bei einem kreisringförmigen Rahmen) zum Rahmen hin dehnen, da die Führungen sich nicht gegenseitig behindern. Sie befinden sich einschließlich ihrer eventuellen weiteren Module und Verankerungsmittel außerhalb der zur Dehnung vorgesehenen Hautareale. Somit besteht auch bei der Dehnung der Haut von außen zum Rahmen hin ein unbehinderter Zugang zu dem Hautareal, das gedehnt wird, um zum Beispiel therapeutische oder messtechnische Maßnahmen vorzunehmen.

Im Folgenden wird die Erfindung anhand der beigefügten Zeichnung näher erläutert und in einem Ausführungsbeispiel dargestellt. Es zeigen:
- Figur 1: die Führung eines erfindungsgemäßen Instruments,
- Figur 2: ein auf der Führung sitzendes Modul,
- Figur 3: in Einzeldarstellung das Modul,
- Figur 4: die Verankerungsmittel des Moduls,
- Figur 5: einen axialen Teilschnitt des Instruments mit zwei Modulen mit entgegengesetzt gerichteten Richtgesperren,
- Figuren 6 bis 8: in schematischer Draufsicht verschiedene Verwendungen des Instruments mit zwei Modulen,
- Figuren 9 und 10: in schematischer Draufsicht verschiedene Verwendungen des Instruments mit drei Modulen und
- Figur 11: in schematischer Draufsicht die Verwendung des Instruments mit einem Rahmen.

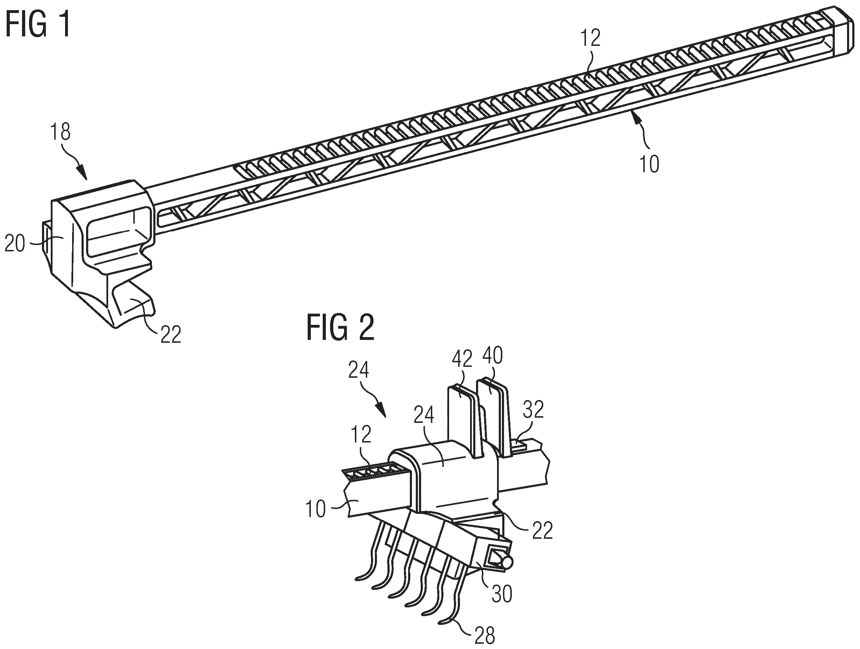

In dem dargestellten Ausführungsbeispiel weist das Instrument zum Dehnen der Haut eine Führung 10 auf, die in Figur 1 als Einzelteil gezeigt ist. Die Führung 10 ist als langgestreckter Stab vorzugsweise aus Kunststoff ausgebildet. Die Führung 10 weist einen unrunden, insbesondere rechteckigen Querschnitt auf. Die Führung 10 ist formstabil, d. h. gegen axiale Zug- und Stauchkräfte sowie gegen Biegekräfte stabil. Im dargestellten Ausführungsbeispiel ist die Führung 10 als gerader Stab ausgebildet. Zur Anpassung an die Körperoberfläche des Patienten kann die Führung gegebenenfalls auch mit einer Längsbiegung ausgebildet sein.

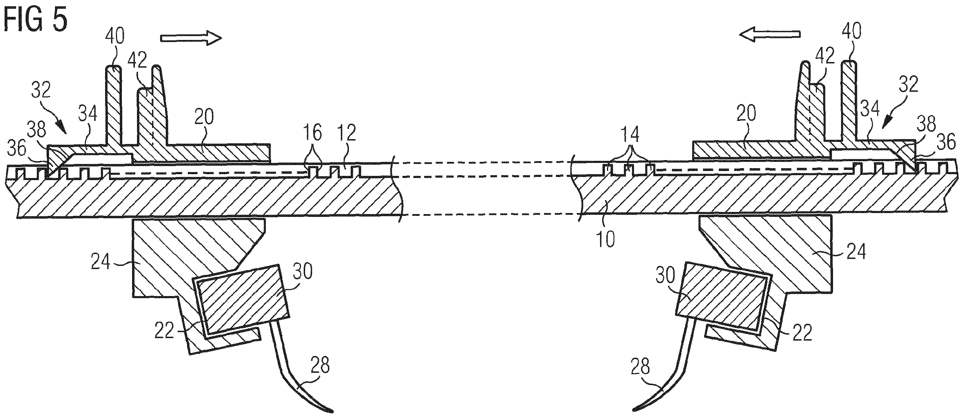

An einer Seitenfläche der Führung 10, in Figur 1 an der Oberseite, ist eine Zahnung 12 ausgebildet, die sich im Wesentlichen über die gesamte Länge der Führung 10 erstreckt. Wie in Figur 5 zu sehen ist, besteht die Zahnung 12 aus in Längsrichtung der Führung 10 beabstandeten Rippen 14, die quer zur Längserstreckung der Führung 10 bzw. der Zahnung 12 verlaufen. Die Rippen weisen 14 weisen im Längsschnitt der Führung 10 einen im Wesentlichen rechteckigen Querschnitt auf, so dass ihre beiderseitigen Flanken jeweils in die entgegengesetzten Längsrichtungen der Führung 10 weisenden Stützflächen 16 bilden, die im Wesentlichen senkrecht zur Längsachse der Führung 10 verlaufen.

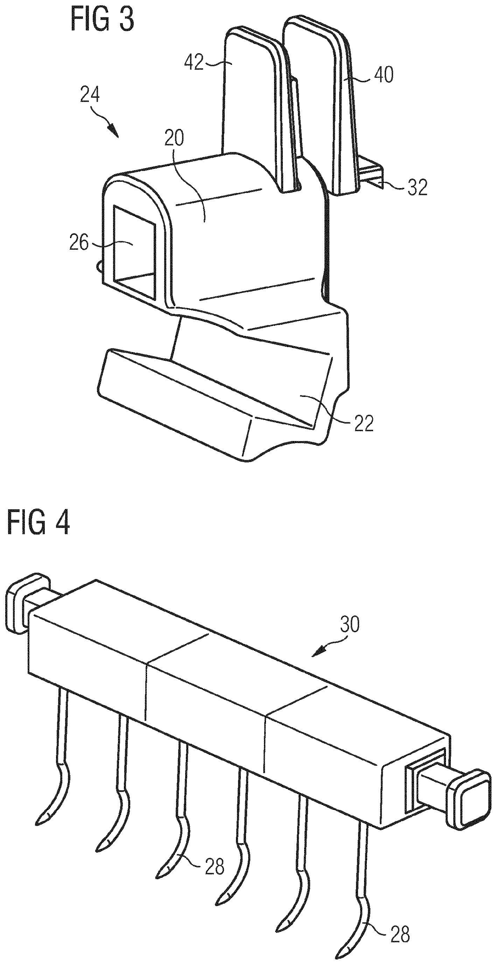

Wie Figur 1 zeigt, ist an dem einen Ende der Führung 10 ein Modul 18 angeordnet, welches im dargestellten Ausführungsbeispiel fest an der Führung 10 angebracht ist, vorzugsweise einstückig zusammen mit der Führung 10 aus Kunststoff geformt ist. Das Modul 18 kann selbstverständlich auch ein separates Bauteil sein, welches auf der Führung 10 sitzt. Dabei kann das Modul 18 gegebenenfalls auch auf der Führung 10 verschiebbar und in seiner Position auf der Führung arretierbar sein. Das Modul 18 weist einen Modulkörper 20 auf und an der Unterseite unterhalb der Führung 10 eine Aufnahme 22, in welche in später erläuterter Weise die Verankerungsmittel einsetzbar sind. Weiter ist auf die Führung 10 wenigstens ein zweites Modul 24 aufgesetzt, dessen Aufbau aus den Figuren 2 bis 5 ersichtlich ist. Das zweite Modul 24 weist einen Modulkörper 20 auf, der mit einem in Längsrichtung durchgehenden Durchbruch 26 ausgebildet ist, dessen Innenquerschnitt dem Außenquerschnitt der Führung 10 entspricht. Das zweite Modul 24 kann mit diesem Durchbruch 26 auf die Führung 10 aufgeschoben werden und ist damit auf der Führung 10 axial verschiebbar und unverdrehbar geführt. An der Unterseite des Modulkörpers 20 sind Verankerungsmittel vorgesehen, die zur Fixierung des Moduls 24 an der Hautoberfläche des Patienten dienen. Die Verankerungsmittel sind in dem dargestellten Ausführungsbeispiel nadelförmige Haken 28, die in die Haut des Patienten eingestochen werden können. Wenigstens zwei Haken 28 sind in einer quer zur Längserstreckung der Führung 10 verlaufenden Reihe nebeneinander angeordnet und sitzen in einem Hakenträger 30, der lösbar in die an der Unterseite des Modulkörpers 20 angeformte Aufnahme 22 einsetzbar ist, wie insbesondere in den Figuren 2 und 5 ersichtlich ist. Das Modul 24 und der Hakenträger 30 sind vorzugsweise ebenfalls aus Kunststoff gefertigt. Die Haken 28 sind Nadeln aus Metall oder Kunststoff.

Anstelle der Haken 28 können an der Unterseite der Module 18 und 24 auch andere Verankerungsmittel vorgesehen sein, um die Module 18 und 24 an der Hautoberfläche des Patienten zu fixieren. Solche Verankerungsmittel können beispielsweise auch adhäsive Mittel oder dergleichen sein. Wesentlich ist, dass die Verankerungsmittel die Einleitung von Kräften in die Haut parallel zur Oberfläche der Haut ermöglichen.

An dem zu der Aufnahme 22 entgegengesetzten Ende des Modulkörpers 20 ist oberhalb des Durchbruchs 26 eine Sperrklinke 32 angeordnet. Im dargestellten Ausführungsbeispiel ist die Sperrklinke 32 als elastisch federnde Zunge 34 aus Kunststoff einstückig an den Modulkörper 20 angeformt. Die Zunge 34 ragt oberhalb der Zahnung 12 und parallel zu dieser von dem Modulkörper 20 ab. Das freie Ende der Zunge 34 ist als Spitze 36 ausgebildet, die von der Zunge 34 nach unten gegen die Führung 10 vorragt und in die Zahnung 12 eingreift, wie in Figur 5 zu sehen ist. Die in die Zahnung 12 eingreifende Spitze 36 ist mit einer Abschrägung 38 ausgebildet, welche von der Unterseite der Zunge 34 gegen die Spitze 36 von dem Modulkörper 20 weg nach unten verläuft, wie Figur 5 zeigt. Die Breite der Zunge 34 mit der Spitze 36 entspricht im Wesentlichen der Breite der Zahnung 12 bzw. die Zunge 34 ist nur wenig schmaler als die Zahnung 12.

An der Oberseite der Zunge 34 ist ein Freigabehebel 40 angeformt, der von der Zunge 34 senkrecht nach oben absteht. Dem Freigabehebel 40 ist ein von dem Modulkörper 20 nach oben abstehend angeformter Anschlaghebel 42 zugeordnet. Zwischen dem Freigabehebel 40 und dem Anschlaghebel 42 bleibt eine axiale Lücke frei.

Die Sperrklinke 32 bildet mit der Zahnung 12 ein Richtgesperre, dessen Funktion aus Figur 5 ersichtlich ist. Ist das Modul 24 auf die Führung 10 aufgeschoben, so greift die Sperrklinke 32 mit der Spitze 36 unter der elastischen Federwirkung der Zunge 34 in die Zahnung 12 ein. Die Spitze 36 befindet sich dabei zwischen zwei aufeinanderfolgenden Rippen 14. Bei dem in Figur 5 linken Modul 24 ist das Richtgesperre so gerichtet, dass das Modul 24 auf der Führung 10 (in Figur 5) nach rechts bewegt werden kann. Dabei wird die Sperrklinke 32 von dem Modul 24 nach rechts gezogen. Die Abschrägung 38 läuft dabei über die Rippe 14. Dadurch wird die Sperrklinke 32 aus der Zahnung 12 herausgehoben und kann über die Zahnung 12 hinweggleiten, so dass das Modul 24 frei bewegt werden kann. Wird das Modul 24 in der entgegengesetzten Sperrrichtung bewegt (bei dem linken Modul 24 in Figur 5 nach links), so schlägt die Spitze 36 der Sperrklinke gegen die senkrechte Stützfläche 16 der nächsten Rippe 14 und blockiert dadurch die Bewegung des Moduls 24.

Erfindungsgemäß können die Bewegungsrichtung und die Sperrrichtung des Richtgesperres umgekehrt werden. Hierzu wird das Modul 24 um 180° gedreht auf die Führung 10 aufgeschoben, wie dies in Figur 5 für das Modul 24 auf der rechten Seite dargestellt ist. In dieser Anordnung kann das Modul 24 in Figur 5 nach links bewegt werden, wobei die Sperrklinke 32 über die Zahnung 12 hinwegläuft, während eine Bewegung in Sperrrichtung, d. h. nach rechts blockiert wird. In Figur 5 sind für beide Stellungen die jeweilige Bewegungsrichtung des Moduls 24 relativ zu der Führung 10 durch einen Pfeil dargestellt.

Selbstverständlich ermöglicht es das Richtgesperre auch in entsprechender Funktionsweise die Führung 10 relativ zu dem festgehaltenen Modul 24 in einer Bewegungsrichtung zu verschieben und in der entgegengesetzten Richtung zu blockieren. Wird z.B. in Figur 5 das linke Modul 24 festgehalten, so kann die Führung 10 in diesem linken Modul 24 nach links bewegt werden, wobei die Führung 10 das rechte Modul 24 mitnimmt.

Wird der Freigabehebel 40 manuell gegen den Anschlaghebel 42 gedrückt, so schwenkt der Freigabehebel 40 die Zunge 34 gegen deren elastische Rückstellkraft hoch und hebt die Spitze 36 aus der Zahnung 12. Dadurch wird das Richtgesperre entriegelt und das Modul 24 kann frei in beiden Richtungen auf der Führung verschoben werden. Dies ist insbesondere erforderlich, um das Modul 24 gegen die Sperrrichtung des Richtgesperres auf die Führung 10 aufschieben zu können und um die auf die Haut ausgeübte Zugkraft aufzuheben.

Es ist ohne Weiteres zu erkennen, dass anstelle des fest mit der Führung 10 verbundenen ersten Moduls 18 auch ein entsprechend dem zweiten Modul 24 ausgebildetes Modul die Funktion des ersten Moduls 18 übernehmen kann. Weiter ist ohne Weiteres ersichtlich, dass zusätzlich zu dem zweiten Modul 24 auch ein entsprechend aufgebautes drittes Modul 44 auf die Führung 10 aufgeschoben werden kann, wobei die Bewegungsrichtung und die Sperrrichtung des zweiten Moduls 24 und des dritten Moduls 44 unabhängig voneinander beliebig umgekehrt werden können.

Wichtig ist bei der Verwendung von Haken 28 diese in Zugrichtung in die Haut einzustechen, um ein Herausrutschen aus der Haut unter der Zugwirkung zu verhindern.

Nachfolgend wird die Verwendung des erfindungsgemäßen Instruments für verschiedene Behandlungsverfahren erläutert.

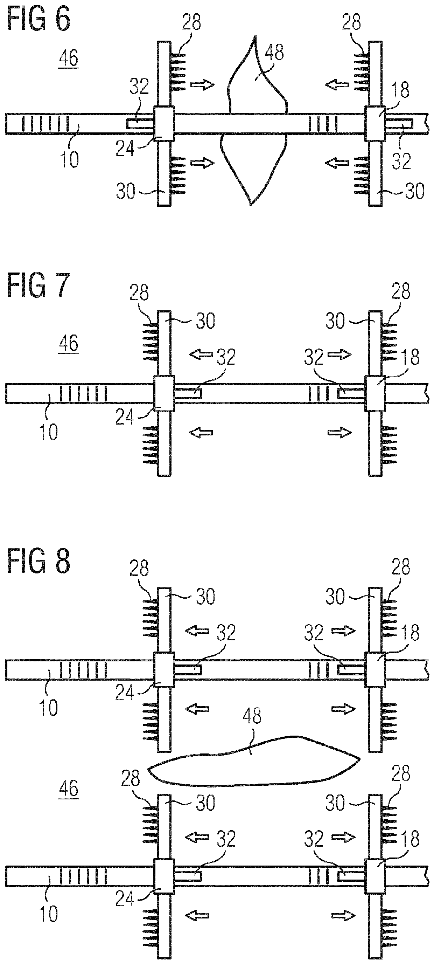

Figur 6 zeigt die Verwendung des Instruments für das bekannte Verfahren der Hautdehnung zum Zwecke eines Wundverschlusses. Dieses Verfahren wird vorzugsweise bei traumatischen Hautdefekten angewendet. Ein erstes Modul 18 und ein zweites Modul 24 werden mit den Verankerungsmitteln in der Haut 46 an den beiden Rändern einer Wunde 48 verankert. Die beiden Module 18 und 24 werden gegeneinander bewegt, wie durch die Pfeile angedeutet ist, um die Haut außerhalb der Module zu dehnen und die Ränder der Wunde 48 zusammenzuziehen, bis diese vernäht werden können. Die Dehnung der Haut außerhalb der an den Wundrändern verankerten Module führt zu einer mechanischen Vergrößerung der Haut und zu einer Reduktion des Gewebeödems, wodurch die Wundränder aneinander angenähert werden können, bis sie durch eine Hautnaht miteinander verbunden werden können.

Figur 7 zeigt, wie durch die erfindungsgemäße Umkehr der Bewegungsrichtung des Moduls 24 die beiden Module 18 und 24 auseinander bewegt werden können, wie ebenfalls durch Pfeile angezeigt ist. Die unverletzte Haut 46 zwischen den Verankerungspunkten der Module 18 und 24 kann hierdurch temporär auseinander gezogen werden. Dadurch wird eine Dehnung der dermalen und subdermalen Zellen verursacht, woraus zusätzlich zur mechanisch bedingten Flächenvergrößerung der Haut eine biochemische Stimulation der Gewebeproliferation und Matrixsynthese resultiert. Werden hohe Dehnungskräfte in die Haut eingeleitet, so kann gezielt eine kurzzeitige Ischämie erzeugt werden, die bei Entspannung der Haut eine vorteilhafte Hyperperfusion zur Folge hat. Eine durch sehr hohe Dehnungskräfte verursachte dosierte Verletzung des dermalen Fasersystems setzt Heilungskräfte in Gang, die zu einem Neuaufbau des Fasersystems und zur Regeneration von Körpergewebe führen (Remodeling).

In Figur 8 ist gezeigt, wie die in Figur 7 erläuterte Hautdehnung zur Behandlung einer chronischen Wunde 48 verwendet werden kann. Zwei Instrumente werden an den beiden Seiten einer chronischen Wunde in der Haut 46 verankert und die Haut 46 wird in diesen an die Wunde angrenzenden Bereichen durch Auseinanderziehen der Module gedehnt. Dadurch wird eine Hautvermehrung in den an die Wunde 48 angrenzenden Bereichen erzielt, die dann zu einem Verschließen der Wunde genutzt werden kann.

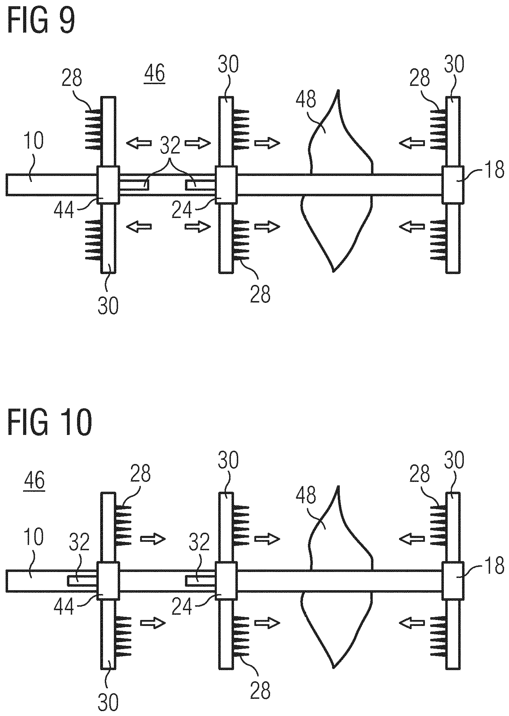

In den Figuren 9 und 10 ist die Verwendung des Instruments mit drei Modulen 18, 24 und 44 dargestellt.

Bei dem in Figur 9 gezeigten Verfahren werden das erste Modul 18 und das zweite Modul 24 in den einander gegenüberliegenden Rändern der Wunde 48 in der Haut verankert. Die Module 18 und 24 werden aufeinander zubewegt, um die Wundränder einander anzunähern. Dabei wird die Haut 46 außerhalb der Module 18 und 24 gedehnt. Ein drittes Modul 44 wird außerhalb des zweiten Moduls 24 auf die Führung 10 aufgesetzt, wobei die Bewegungsrichtung und die Sperrrichtung entgegengesetzt zu dem zweiten Modul 24 angeordnet sind. Die Haut außerhalb des zweiten Moduls 24 kann dadurch mit Hilfe des dritten Moduls 44 zusätzlich gedehnt werden. Dadurch wird in einem zusätzlichen Hautareal die Ausschüttung von biochemischen Wachstumsfaktoren getriggert, die durch eine Stimulation der Gewebeneubildung und Wundheilung regenerative Vorgänge in Gang setzen und optimieren (s. Figur 7).

In Figur 10 werden ebenfalls das erste Modul 18 und das zweite Modul 24 in den einander gegenüberliegenden Wundrändern verankert, um diese Wundränder zusammenzuziehen. Das dritte Modul 44 ist außerhalb des zweiten Moduls 24 auf die Führung 10 aufgesetzt. Dabei ist allerdings die Bewegungsrichtung und die Sperrrichtung des dritten Moduls 44 gleich gerichtet wie bei dem zweiten Modul 24. Das dritte Modul 44 kann dadurch die Haut 46 auf der von der Wunde 48 abgewandten Seite des dritten Moduls 44 zusätzlich dehnen, so dass durch das dritte Modul 44 die zusätzlich stimulierte Haut zu dem zweiten Modul 24 und damit zu der Wunde 48 nachgeschoben wird.

Durch die Erfindung ist es möglich, mit demselben Instrument sowohl eine Wundkontraktion für den Verschluss einer Wunde als auch eine Hautdistraktion zur Stimulation der Zellproliferation und Matrixsynthese durchzuführen. Dabei ist es insbesondere auch möglich, mittels des Instruments diese beiden Funktionen miteinander zu kombinieren.

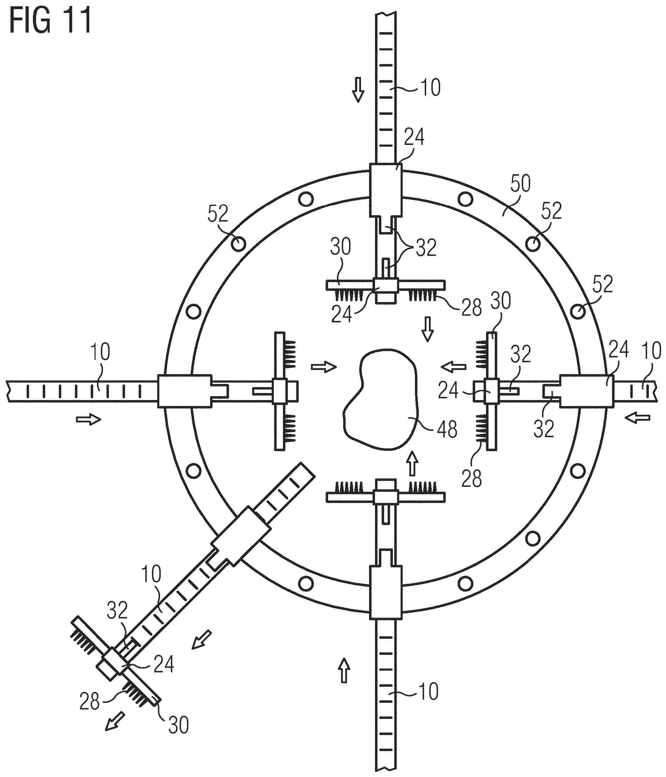

Eine weitere Verwendung des erfindungsgemäßen Instruments ist in der Figur 11 dargestellt.

In dieser Ausführung wird ein starrer Rahmen 50 verwendet, der vorzugsweise ringförmig ausgebildet ist und die Form eines Rechtecks, eines Mehrecks oder auch eines Kreisrings aufweisen kann, wie dies in der Zeichnung dargestellt ist. Der ebene Rahmen 50 weist einen freien Innenraum auf, in welchem sich bei der Anwendung die zu behandelnde Wunde 48 befindet. Auf dem Rahmen 50 können ein oder mehrere Module 24 angeordnet werden. Vorzugsweise werden die Module 24 auf dem Rahmen in variablen Positionen fixiert. Hierzu kann der Rahmen 50 beispielsweise im Umfangswinkel gegeneinander versetzte Bohrungen 52 aufweisen, in welche die Module 24 mit an ihrer Unterseite angeformten Zapfen eingesetzt werden. Auf diese Weise können die Module 24 auf dem Rahmen 50 in umkehrbarer Anordnung ausgerichtet werden. Die in die Module 24 eingeführten Führungen 10 sind auf diese Weise in den an dem Rahmen 50 fixierten Modulen 24 relativ zu den Modulen 24 und dem Rahmen 50 bewegbar. Je nach Anordnung der Module 24 in dem Rahmen 50 kann das Richtgesperre dabei so wirken, dass die Führungen 10 in der Bewegungsrichtung nach innen in den Innenraum des Rahmens 50 bewegt werden können, während ihre Bewegung nach außen blockiert wird. Bei Umkehrungen des Richtgesperres wird eine Bewegung der Führung 10 relativ zu dem Rahmen 50 nach außen ermöglicht, während die Bewegung in den Innenraum des Rahmens 50 blockiert ist.

Auf den Führungen 10 können jeweils ein oder mehrere weitere Module 24 angeordnet sein. Diese Module können als fest an der Führung angeordnete Module 18 oder als mittels eines Richtgesperres in einer Richtung auf der Führung 10 verschiebbare Module 24 ausgebildet sein, wie dies vorstehend beschrieben ist.

Auf Grund der unterschiedlichen Positionen der Module 24 auf dem Rahmen 50 verlaufen die Bewegungsrichtungen der Führungen 10 in diesen jeweiligen Modulen 24 in der Ebene des Rahmens 50 in unterschiedlichen Winkeln zueinander. Wie dies in Figur 11 zu sehen ist können die Bewegungsrichtungen in Winkeln von z. B. 30°, 60°, 90°, 180° usw. verlaufen. Je nach der Ausrichtung des Richtgesperres des jeweils auf dem Rahmen 50 fixierten Moduls 24 kann ein weiteres innerhalb des Rahmens 50 auf der Führung 10 sitzendes Modul 24 nach innen in den Innenraum des Rahmens 50 geschoben werden bzw. ein außerhalb des Rahmens 50 auf der jeweiligen Führung 10 sitzendes Modul 24 kann radial außen von dem Rahmen 50 wegbewegt werden. Dies ist in Figur 11 jeweils durch Pfeile angezeigt. Werden innerhalb des Rahmens 50 angeordnete Module 24 in der Haut verankert und mittels der Führungen 10 radial in den Innenraum des Rahmens 50 geschoben, so können durch diese Module 24 die Ränder einer von dem Rahmen 50 umschlossenen Wunde 48 gegeneinander geschoben werden, um die Haut am Rand der Wunde 48 zu dehnen und die Wunde 48 zu schließen, wie dies beispielsweise in den Figuren 6 und 8 erläutert ist. Wird ein zweites Modul 24 außerhalb des Rahmens 50 auf der Führung 10 angeordnet und das Richtgesperre des auf dem Rahmen 50 fixierten Moduls 24 ist so ausgerichtet, dass die Führung 10 radial nach außen geschoben werden kann, so ist eine zusätzliche Dehnung der Haut außerhalb des Rahmens 50 möglich, um einen zusätzlichen Hautgewinn zu erzielen, wie dies z. B. anhand der Figur 7 erläutert ist.

Bei allen diesen Verwendungen ergibt sich der Vorteil, dass der Rahmen 50 und insbesondere die Führungen 10 mit den Modulen 24 außerhalb des zu behandelnden Hautareals und der Wunde 48 liegen und den freien Zugang zu dem zu behandelnden Hautareal und zu der Wunde 48 nicht behindern oder beschränken. Dieser Vorteil bleibt auch dann erhalten, wenn mehrere Instrumente auf dem Rahmen 50 angeordnet sind, wie dies z. B. in Figur 11 dargestellt ist, und dadurch eine Hautdehnung an dem gesamten Umfang der Wunde 48 mit unterschiedlichen Zugrichtungen möglich ist.

### Bezugszeichenliste

- 10: Führung
- 12: Zahnung
- 14: Rippen
- 16: Stützfläche
- 18: erstes Modul

- 20: Modulkörper
- 22: Aufnahme
- 24: zweites Modul
- 26: Durchbruch
- 28: Haken

- 30: Hakenträger
- 32: Sperrklinke
- 34: Zunge
- 36: Spitze
- 38: Abschrägung

- 40: Freigabehebel
- 42: Anschlaghebel
- 44: drittes Modul
- 46: Haut
- 48: Wunde

- 50: Rahmen
- 52: Bohrungen

## Patentansprüche

1. Instrument zum Dehnen der Haut, mit einer langgestreckten formstabilen Führung (10), mit wenigstens zwei auf der Führung (10) angeordneten Modulen (18, 24, 44), mit Verankerungsmitteln zur Fixierung wenigstens eines der Module (18, 24, 44) an der Haut, wobei wenigstens eines der Module (24) und die Führung (10) in deren Längsrichtung relativ zueinander bewegbar sind und wobei ein Richtgesperre diese Bewegung in einer Bewegungsrichtung ermöglicht und in der entgegengesetzten Sperrrichtung blockiert,
**dadurch gekennzeichnet, dass** das Richtgesperre bezüglich der Bewegungsrichtung und der Sperrrichtung umkehrbar ist.

2. Instrument nach Anspruch 1, wobei wenigstens drei Module (18, 24, 44) auf der Führung (10) angeordnet sind, dass wenigstens zwei dieser Module(24, 44) relativ zu der Führung (10) bewegbar sind und ein Richtgesperre aufweisen, und dass das Richtgesperre wenigstens eines dieser Module (24, 44) umkehrbar ist.

3. Instrument nach Anspruch 1 oder 2, wobei das Richtgesperre eine an der Führung (10) ausgebildete in Längsrichtung der Führung (10) verlaufende Zahnung (12) und wenigstens eine an dem Modul (24) angeordnete Sperrklinke (32) aufweist, wobei die Zahnung (12) aus quer zur Längsrichtung verlaufenden Rippen (14) besteht, die jeweils an ihren beiden in Längsrichtung weisenden Stirnflächen mit senkrecht zur Längsrichtung angeordneten Stützflächen (16) ausgebildet sind, wobei die Sperrklinke (32) mit einer elastisch auslenkbaren freien Spitze (36) in die Zahnung (12) eingreift, wobei die Spitze (36) eine Abschrägung (38) aufweist, die bei Bewegung in der Bewegungsrichtung über die Rippen (14) läuft, wodurch die Sperrklinke (32) gegen ihre elastische Rückstellkraft aus der Zahnung (12) ausgehoben wird, und wobei die Spitze (36) der Sperrklinke (32) bei Bewegung in Sperrrichtung gegen die Stützfläche (16) der Rippen (14) läuft.

4. Instrument nach Anspruch 3, wobei die Sperrklinke (32) an dem in der Bewegungsrichtung nachlaufenden Ende des Moduls (24) angeordnet ist und dass das Modul (24) zur Umkehr des Richtgesperres in zwei um 180° gedrehten Ausrichtungen auf die Führung (10) aufschiebbar ist.

5. Instrument nach einem der vorhergehenden Ansprüche, wobei das Richtgesperre mittels einer Freigabeeinrichtung (40) außer Funktion gebracht werden kann.

6. Instrument nach Anspruch 3 oder 4, wobei eine Freigabeeinrichtung (40) die Sperrklinke (32) gegen ihre elastische Rückstellkraft aus der Zahnung (12) heraushebt.

7. Instrument nach einem der vorhergehenden Ansprüche, wobei wenigstens zwei Führungen (10) an einem starren Rahmen (50) angeordnet sind, dass die Bewegungsrichtung dieser Führungen (10) in der Ebene des Rahmens (50) in einem Winkel zueinander verlaufen und dass ein auf der jeweiligen Führung (10) angeordnetes Modul (24) an dem Rahmen (50) fixierbar ist, sodass die Führung (10) bezüglich dieses Moduls (24) und bezüglich des Rahmens (50) in ihrer Längsrichtung bewegbar ist.

8. Instrument nach Anspruch 7, wobei der Rahmen (50) ein in einer Ebene ringförmig geschlossener Rahmen (50) ist, der eine freie Innenfläche umschließt.

## Claims

1. Instrument for stretching skin with an elongated, dimensionally stable track (10), with at least two modules (18, 24, 44) arranged on the track (10), with anchoring means for fixating at least one of the modules (18, 24, 44) onto the skin, wherein at least one of the modules (24) and the track (10) are moveable relative to each other in their longitudinal directions and wherein a directional ratchet allows this movement in one direction of movement and blocks the movement in the opposite direction, **characterized in that** the directional ratchet is reversible with regard to the direction of movement and the direction of locking.

2. Instrument in accordance with claim 1,
wherein at least three modules (18, 24, 44) are arranged on the track (10), at least two of these modules (24, 44) are moveable relative to the track (10) and comprise a directional ratchet and wherein the directional ratchet of at least one of these modules (24, 44) is reversible.

3. Instrument in accordance with claim 1 or 2,
wherein the directional ratchet comprises a toothing (12), implemented in the longitudinal direction of the track (10), and at least one pawl (32) arranged on the module (24), wherein the toothing (12) is comprised of ribs (14), extending perpendicular to the longitudinal direction, each of which are implemented with bearing surfaces (16), arranged vertically to the longitudinal direction on each of the respective end faces that faces in the longitudinal direction, wherein the pawl (32) engages with the toothing (12) by means of a deflectable elastic free tip (36), wherein the tip (36) comprises a sloping edge (38) which, when moved, moves over the ribs (14) in the direction of movement, whereby the pawl (32) is lifted, counter to its elastic return force, out of the toothing (12), and wherein the tip (36) of the pawl (32) moves against the bearing surface (16) of the ribs (14) when moved in the locking direction.

4. Instrument in accordance with claim 3,
wherein the pawl (32) is arranged on the end of the module (24) that trails in the direction of movement and the module (24) can be slid onto the track (10) in two directions, turned at 180°, in order to reverse the directional ratchet.

5. Instrument in accordance with any of the preceding claims,
wherein the directional ratchet can be taken out of operation by means of a releasing device (40).

6. Instrument in accordance with claim 3 or 4,
wherein the releasing device (40) lifts the pawl (32), counter to its elastic return force, out of the toothing (12).

7. Instrument in accordance with any of the preceding claims,
wherein at least two tracks (10) are arranged on a rigid frame (50), the movement of direction of these tracks (10) extend at an angle to each other on the plane of the frame (50), and a module (24) arranged on each of the tracks (10) can be fixated on the frame (50) such that the track (10) is moveable in its longitudinal direction with respect to this module (24) and with respect to the frame (50).

8. Instrument in accordance with claim 7,
wherein the frame (50) is a frame which is circularly closed on one plane and which encloses a free inner surface.

## Revendications

1. Instrument pour tendre la peau, comprenant un guide allongé (10) de forme stable, au moins deux modules (18, 24, 44) installés sur le guide (10), des moyens d'ancrage pour fixer au moins l'un des modules (18, 24, 44) à la peau,
- au moins l'un des modules (24) et le guide (10) étant mobiles l'un par rapport à l'autre dans la direction longitudinale, et
- un verrou directionnel permettant ce mouvement dans une direction de mouvements et bloquant ce mouvement dans la direction de blocage, opposée,
instrument **caractérisé en ce que**
le verrou directionnel est réversible entre la direction de mouvement et la direction de blocage.

2. Instrument selon la revendication 1,
dans lequel
au moins trois modules (18, 24, 44) sont installés sur le guide (10),
- dont au moins deux de ces modules (24, 44) sont mobiles par rapport au guide (10) et comportent un verrou directionnel, et
le verrou directionnel d'au moins l'un des modules (24, 44) est réversible.

3. Instrument selon la revendication 1 ou 2,
dans lequel
le verrou directionnel comporte des dents (12) réalisées sur le guide (10) dans la direction longitudinale du guide (10) et au moins un cliquet de blocage (32) sur le module (24),
les dents (12) se composant de nervures (14) orientées transversalement à la direction longitudinale et réalisant respectivement sur leurs deux surfaces frontales tournées dans la direction longitudinale, des surfaces d'appui (16) perpendiculaires à la direction longitudinale,
le cliquet de blocage (32) ayant une pointe libre (36) qui se dévie élastiquement pour pénétrer dans les dents (12),
la pointe (36) ayant une surface inclinée (38) qui, lors du mouvement dans la direction de mouvement passe sur les nervures (14) de façon que le cliquet (32) se dégage des dents (12) contre sa force de rappel élastique, et
la pointe (36) du cliquet (32) vient contre la surface d'appui (16) des nervures (14) pour un mouvement dans la direction de blocage.

4. Instrument selon la revendication 3,
**caractérisé en ce que**
le cliquet (32) est à l'extrémité aval du module (24) selon la direction de mouvement et le module (24) peut être emmanché sur le guide (10) dans deux directions tournées de 180° pour inverser le verrou directionnel.

5. Instrument selon l'une des revendications précédentes,
dans lequel
le verrou directionnel se neutralise par un dispositif de libération (40).

6. Instrument selon la revendication 3 ou 4,
dans lequel
le dispositif de libération (40) dégage le cliquet (32) des dents (12) contre sa force de rappel élastique.

7. Instrument selon l'une des revendications précédentes,
dans lequel
au moins deux guides (10) sont fixés à un châssis rigide (50) et la direction de mouvement de ces guides (10) dans le plan du cadre (50) est orientée suivant un angle, l'une par rapport à l'autre, et un module (24) installé sur le guide respectif étant fixé au cadre (50) pour que le guide (10) soit mobile dans sa direction longitudinale par rapport à ce module (24) et à ce cadre (50).

8. Instrument selon la revendication 7,
dans lequel
le cadre (50) est un cadre (50) fermé de forme annulaire dans un plan et laissant une surface intérieure dégagée.
